(19) Europäisches Patentamt
European Patent Office
Office européen des brevets

(11) **EP 1 986 706 B1**

(12) **EUROPEAN PATENT SPECIFICATION**

(45) Date of publication and mention
of the grant of the patent:
**17.08.2011 Bulletin 2011/33**

(21) Application number: **07710404.0**

(22) Date of filing: **30.01.2007**

(51) Int Cl.:
*A61L 24/00* (2006.01)    *A61L 24/06* (2006.01)

(86) International application number:
**PCT/US2007/061325**

(87) International publication number:
**WO 2007/090130 (09.08.2007 Gazette 2007/32)**

(54) **POROUS INTRAVASCULAR EMBOLIZATION PARTICLES AND METHODS OF MAKING THEM**

PORÖSE INTRAVASKULÄRE EMBOLISIERUNGSTEILCHEN UND VERFAHREN ZU DEREN HERSTELLUNG

PARTICULES D'EMBOLISATION INTRAVASCULAIRES POREUSES  ET  LEURS PROCÉDÉS DE FABRICATION

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(30) Priority: **30.01.2006  US 763657 P
30.01.2006  US 763656 P**

(43) Date of publication of application:
**05.11.2008  Bulletin 2008/45**

(60) Divisional application:
**11154646.1**

(73) Proprietor: **BIOSPHERE MEDICAL, INC.
Rockland, MA 02370 (US)**

(72) Inventors:
• **MCNAMARA, Gerald, R.
Reno, NV 89509 (US)**
• **BRANDOM, Donald, K.
Davis, CA 95616 (US)**
• **BALKENHOL, Wayne, J.
Los Altos Hills, CA 94022 (US)**
• **MATSON, Louis, R.
Pollock Pines, CA 95726 (US)**

(74) Representative: **Bradley, Adrian
Cleveland
10-14 Duke Street
Reading
Berkshire RG1 4RU (GB)**

(56) References cited:
EP-A- 0 256 293      EP-A- 0 448 391
WO-A-03/084582      WO-A-2006/046155
US-A1- 2003 059 371

**Description**

The present invention relates to the use of porous embolization particles and methods of making them.

Intravascular interventional procedures produce artificial embolization that can be useful in mammals for controlling internal bleeding, blocking blood supply to tumors or relieving pressure In vessel walls near aneurysms. Known methods for providing an artificial embolism include use of (1) inflatable and detachable balloons, (2) coagulative substances, (3) later curing polymeric substances, (4) occlusive wire coils, and (5) embolization particles.

One type of embolization particle is made of polyvinyl alcohol (PVA). Though there are several methods for manufacturing crosslinked PVA foam embolization particles, the basic method includes mixing a PVA solution with acid, formalin, water, and air or starch or polyethylene glycol to provide the foam structure; then reacting the mixture for a period of time at elevated temperatures until the polyvinyl alcohol is crosslinked to form a PVA sponge product. Subsequently, the residual formalin and other processing aids are rinsed out, the material is chopped or ground into fine particles, and then the particles are dried and separated into several size ranges using sieve-sizing. Finally, the resulting product is packaged and sterilize for use. In one alternative variation of the above process, the crosslinking occurs in a secondary bath by dropping the reaction mixture into the bath for formation of spheres of various sizes, where they are cured into their final shape.

WO 03/084582 relates to an embolic composition comprising substantially spherical embolic particles having a diameter of about 1200 micron or less, the particles comprising polyvinyl alcohol, and including an interior having relatively large pores and a surface region having fewer relatively large pores. US 2003/0059371 relates to a composition for forming an occlusion in a body opening or cavity comprising hydrated, partially acetalized, polyvinyl alcohol foam particles having particle size, pore size, and particle porosity, the particles being substantially suspended in a selected injectable, biologically acceptable, liquid medium having a liquid medium specific gravity.

Traditional non-spherical PVA foam or sponge embolization particles are irregularly shaped and generally contain a range of pore sizes that are produced during the manufacturing process by whipping air into the PVA solution prior to crosslinking. Disadvantages of these particles include their non-precise size (aspect ratios) and open edges on the particles that cause them to clump together and subsequently plug up delivery catheters or occlude at a site proximal to the target site.

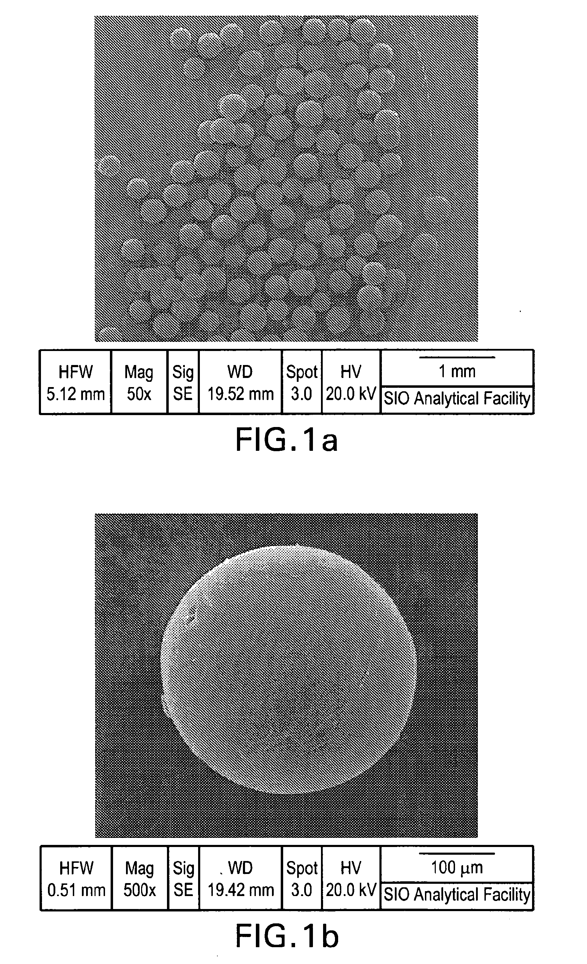

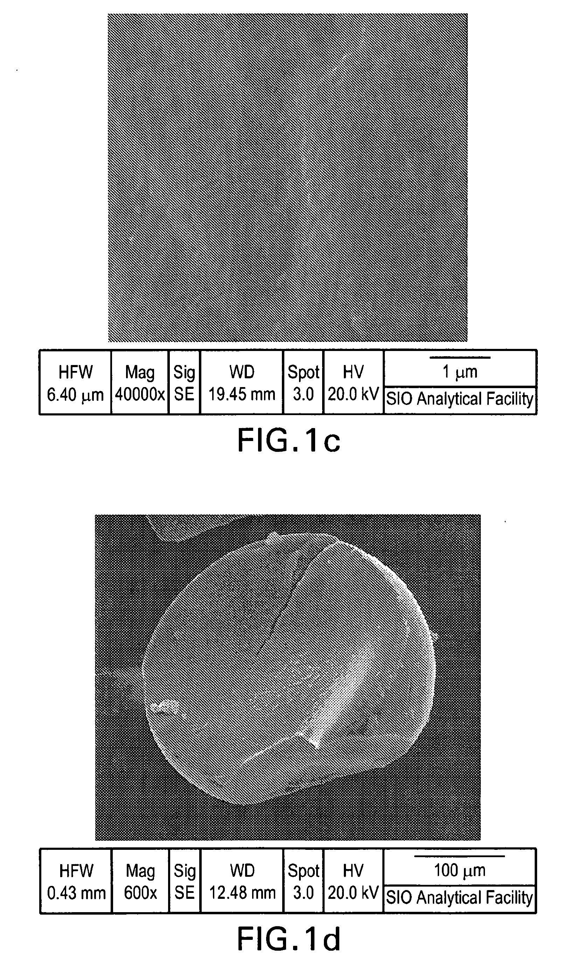

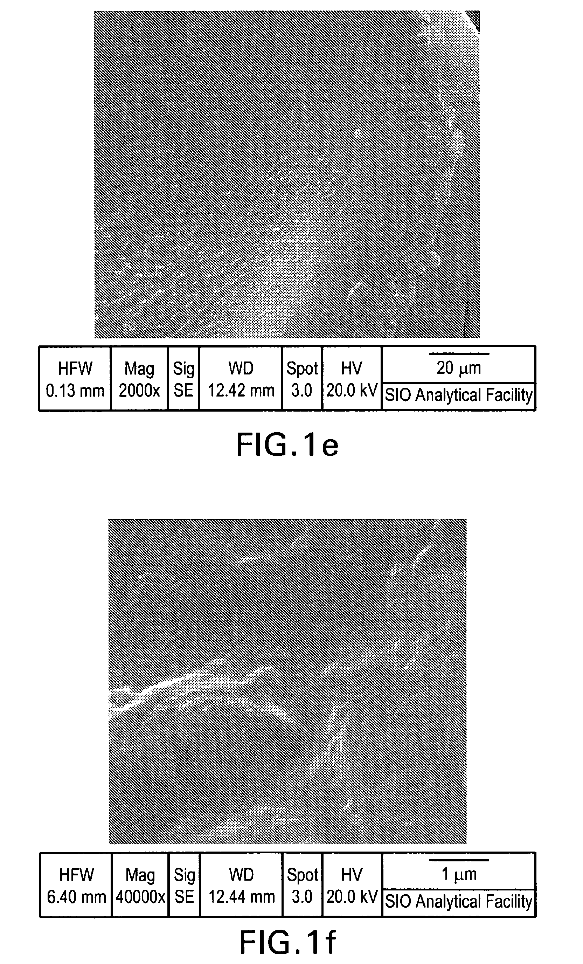

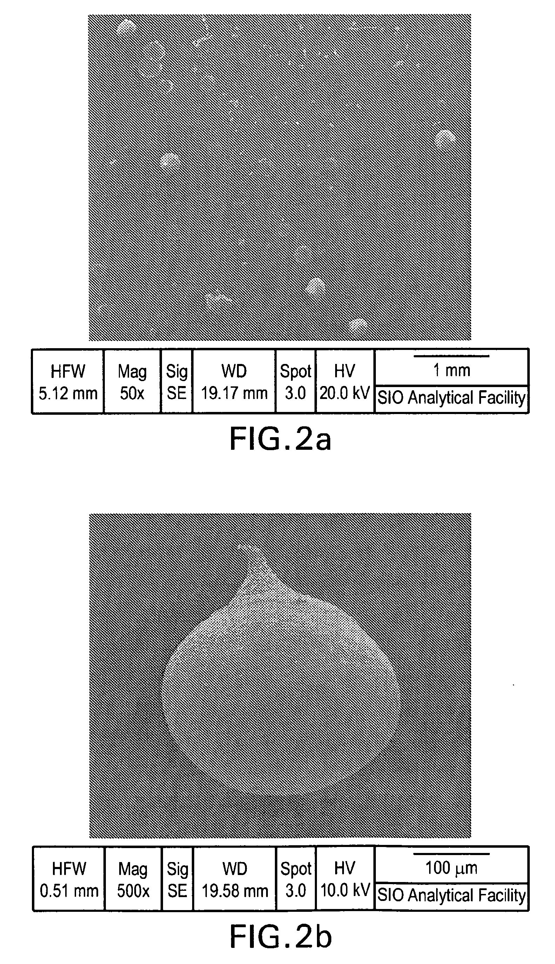

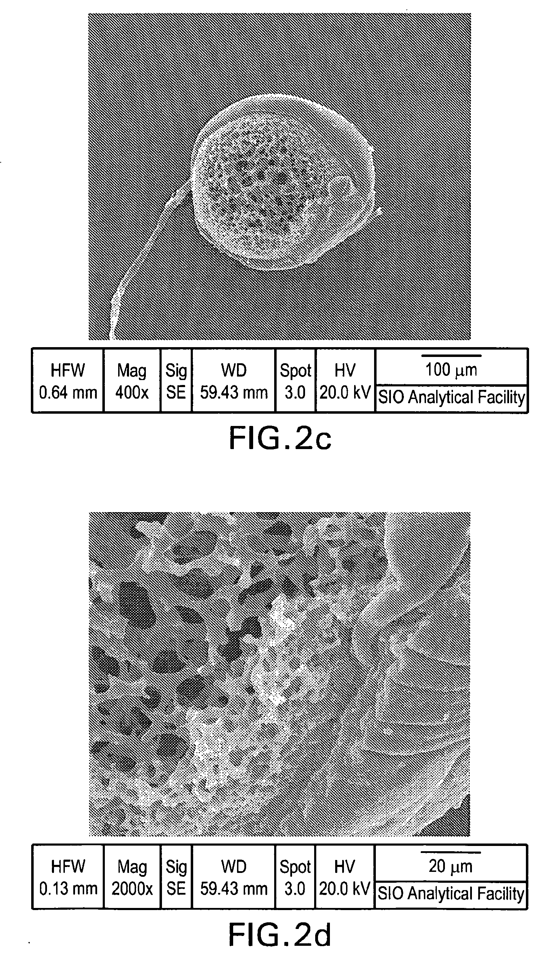

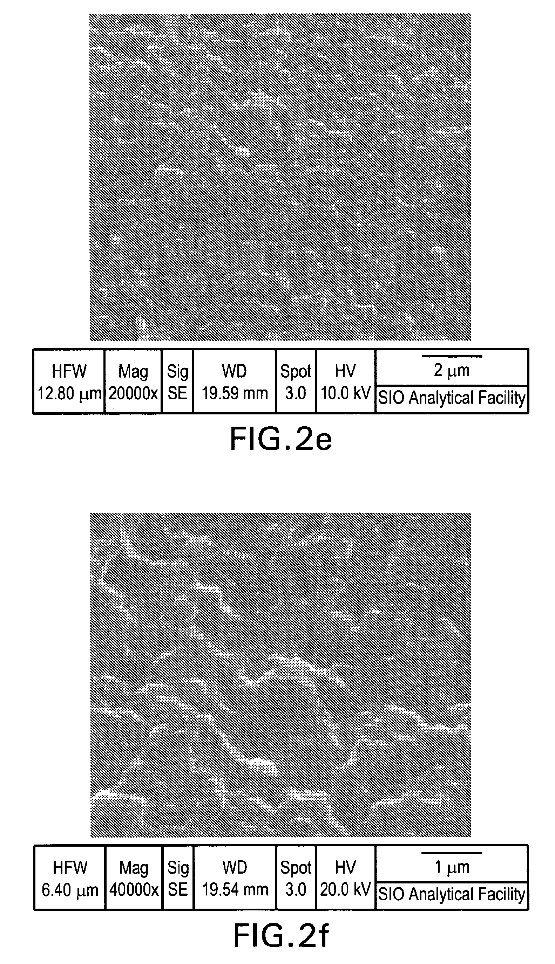

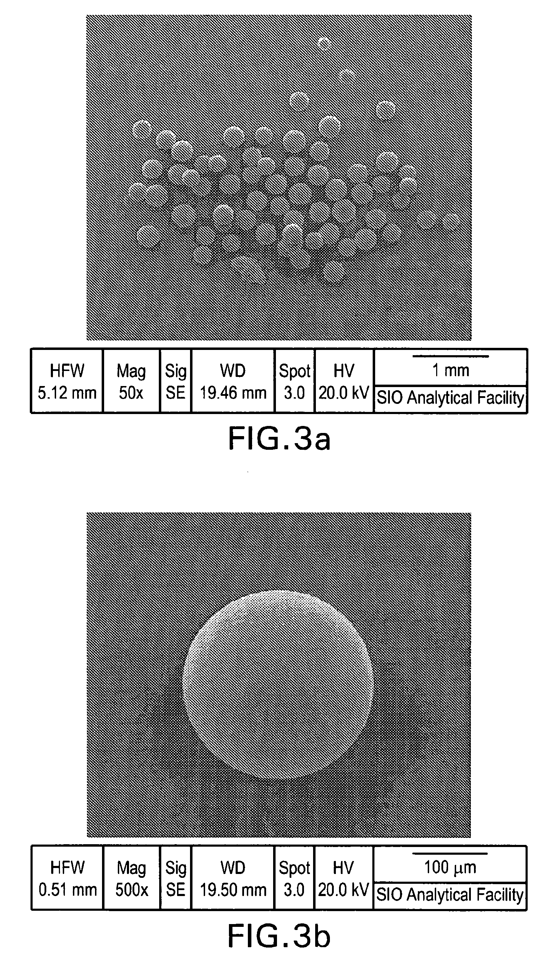

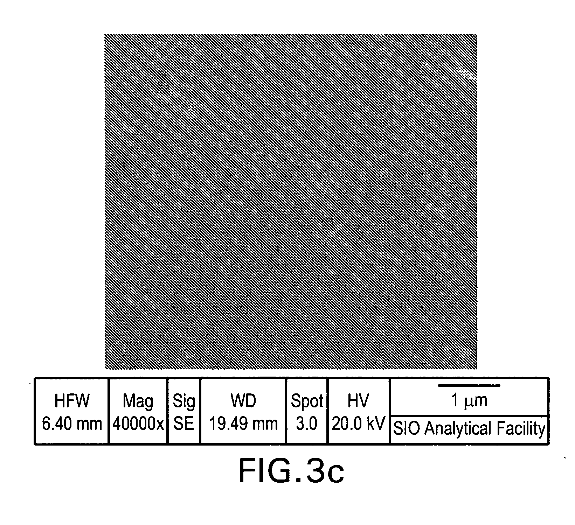

Spherical particles minimize these disadvantages. Spherical particles can penetrate deeper into the vasculature than traditional particles due to the uniform shape of the particle. They are reported to infrequently occlude delivery catheters. Existing spherical embolies include Biocompatibles International plc's Bead Block™ (FIG. 3), Biosphere Medical, Inc.'s Embosphere™ (FiG. 1) and Boston Scientific Corporation's Contour SE™ (FIG. 2). The Bead Block™ product is PVA gel rather than foam and does not have macropores (that is, the pores are less than 1 micron in diameter). Embosphere™ is a gel made of an acrylic co-polymer (trisacryl) and does not have macropores. Contour SE™ is made of PVA, has an onion shape but has no surface macropores.

However, spherical embolization particles known in the art have several disadvantages. For example, the smooth surface of these particles may affect the stable integration of such particles within the occlusive mass comprising the particles, clotted blood and ultimately fibrous tissue. In addition, the compression and elastic recovery properties of such particles may allow undesirable migration under the pressure within the blood vessel at the embolization site. The particles described above are delivered to the user in a pre-hydrated state (typically in a saline solution). Subsequently, they must be mixed with contrast agent by the user prior to use for a time sufficient for the particles to equilibrate in the mixture of saline and contrast agent ("contrast media"). That is, the pre-hydrated particles in the prior art are typically provided in saline, which has a specific gravity that is close to 1.0. However, when the particles, which contain the saline within their structure (or polymeric matrix), are placed in the contrast media (which has a specific gravity of about 9.0 to 1.5), the particles containing the saline within their structure float to the top of the contrast media until they have equilibrated to the new solution conditions. In addition, while hydrated particles are available, such products have shelf-life limitations and are more expensive to manufacture than dry particles because they require aseptic liquid processing.

A further disadvantage is that many of these known spherical particles cannot be dehydrated to from a dry particle. That is, the particles must be provided in a prehydration fluid of some kind in order to be used. More specifically, the Contour SE™ product, once dehydrated, cannot be re-hydrated with equipment typically available in an interventional radiology procedure room. Further, in the case of Embosphere™, the particles physically crack upon dehydration. The particles may upon dehydration also permanently deform substantially from their spherical shape.

The invention is directed to improved porous embolization particles and methods of making them.

In one embodiment, the present invention relates to a dehydrated substantially spherical embolization particle for use in a method of embolization, wherein the particle comprises a crosslinked polymer, wherein the interior of said particle comprises interconnected pores that extend to the surface of the particle, wherein at least 20% of the pores are interconnected, wherein the surface pores have an average diameter of greater than 1 $\mu m$ (micron), and wherein the particle has a diameter ranging from 45 to 1400 $\mu m$ (microns); and wherein the method of embolization comprises the steps of rehydrating the dehydrated particle, and positioning the rehydrated particle in a target region of a blood vessel.

In a further embodiment, the present invention relates to an embolization device comprising (i) a catheter, and (ii) the embolization particle of the invention.

In a further embodiment, the present invention relates to a container comprising the particle of the invention, wherein the polymer comprises polyvinyl alcohol.

In a further embodiment, the present invention relates to a kit comprising the container of the invention, and further comprising at least one of (i) a container of hydration fluid, (ii) an apparatus for reducing pressure during hydration, and (iii) instructions for preparation and/or application of the particles.

In a further embodiment, the particle of the invention is for use in occlusion of blood vessels by embolization, or for use in the treatment of aneurysms, tumors, bleeding, or vascular malformations by embolization.

In a further embodiment, the present invention relates to a method of producing the particle of the invention, wherein the polymer comprises polyvinyl alcohol, the method comprising: (a) mixing polyvinyl alcohol and deionized water, and heating; (b) adding porogen to the mixture of (a), (c) adding a crosslinking agent and a crosslinking reactant to the mixture of (b), and mixing; (d) degassing the mixture of (c); (e) adding the degassed mixture of (d) drop-wise to a medium comprising a surfactant and a hydrophobic material; (f) stirring the medium to obtain substantially spherical particles; and (g) dehydrating the substantially spherical particles.

Dry and hydrated porous embolization particles are disclosed that are substantially spherical. The particle has a surface portion and an interior portion. The interior contains pores, many of which are interconnected. Some of the pores are exposed to the surface of the particles. The surface pores have a diameter of greater than 1 micron. The particle is made of a polymer and has a diameter ranging from 45 microns to 1400 microns in its dehydrated form.

The embolization particles have a firmness not found in the prior art spherical embolization particles. In some embodiments, the particle comprises a crosslinked PVA polymer foam that defines a plurality of pores In the particle. The pores have an average diameter of greater than 1 micron and the pores disposed on the surface of the particle, for the most part, are in communication with the pores disposed within the particle. The PVA particle is substantially spherical and has a diameter of 45 microns or more.

The embolization particle is for use in a method of embolization. The method includes positioning embolization particles in a target region of a blood vessel. The particles are of the type described herein.

The embolization particles can be made by mixing a PVA solution and a porogen, adding a crosslinking agent, centrifuging the mixture to remove air bubbles, adding the mixture to a reaction medium and stirring the mixture and medium to form crosslinked substantially spherical porous embolization particles. The particles are then dried and sized by sieving to form a dehydrated embolization particle.

The embolization particles can be rehydrated prior to use by suspending the particles in a saline solution with or without contrast agent in a sealed device, whereby entrapped gases are removed from the particles. A syringe can be used to reduce the pressure within a sealed device such as a vial to accomplish such removal. Alternatively, the sealed device can be the syringe.

The hydrated embolization particles can then be used directly for embolization at a target region within a blood vessel.

FIG. 1 is scanning electron micrograph (SEM) of the prior art Embosphere™ acrylic hydrogel embolization particles. FIG. 1A shows population of the particles at 50x magnification, while FIG. 1B shows the smooth surface of the particle at 500x magnification. FIG. 1C shows the surface of the particle in FIG. 1B at 40,000x magnification. No macropores greater than 1 micron are shown. FIG. 1D shows a the interior of a cut Embosphere™ embolization particle at 600X magnification. FIG. 1E shows the interior surface of the particle in FIG. 1D at 2000x magnification, while FIG. 1F discloses the interior surface at 40,000x magnification. No macropores are apparent on the interior of these particles.

FIG. 2 depicts SEMs of the Contour SE™ embolization particles. FIG. 2A shows a population of the particles at 50x magnification, while FIG. 2B (insert space here in your master) shows a single particle at 500x magnification. The particles have an onion-like shape and do not contain macropores. FIGS. 2C and D show the interior of a cut particle at 400x and 2000x magnification, respectively. FIGS. 2E and F show close ups of the surface of the particle at different locations at 20,000x and 40,000x, respectively. No macropores are shown.

FIGS. 3A-3C depict SEMs of the Bead Block™ PVA gel embolization particles. FIG. 3A shows a population of the particles at 50X magnification, while FIG. 3B shows a single particle at 500 magnification. No macropores are apparent. FIG. 3C shows the surface of the particle in FIG. 3B at 40,000 magnification. The few pores shown are substantially smaller than 1 micron in diameter.

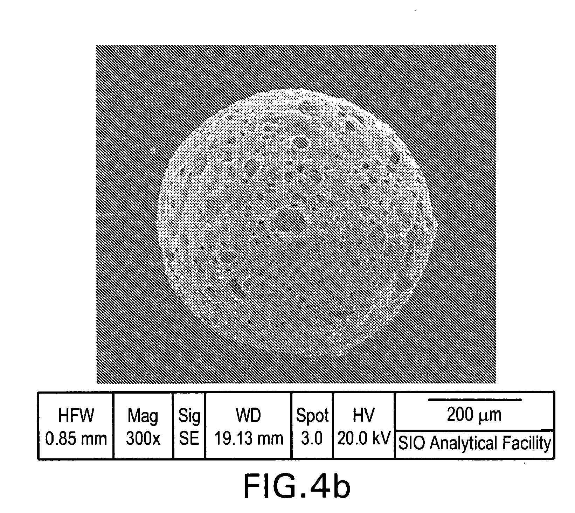

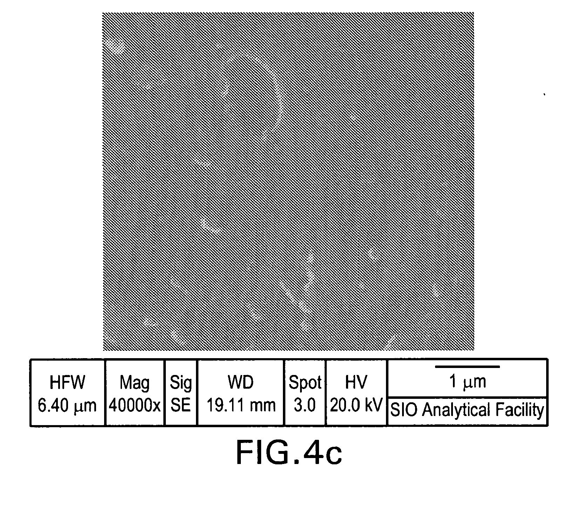

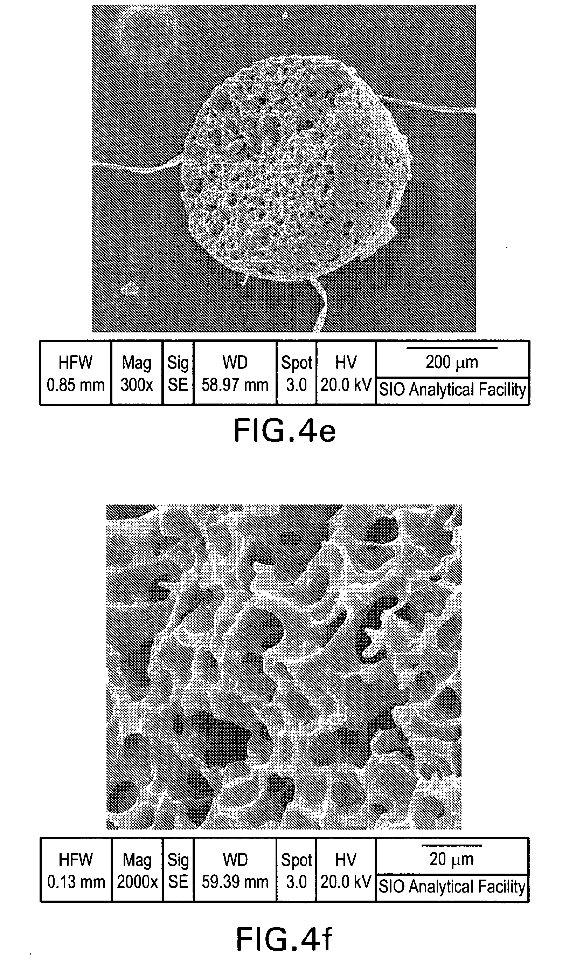

FIGS. 4A through 4F are (SEM's) of PVA embolization particles made according to the protocols described herein. FIG. 4A shows a population of dehydrated PVA particles. As can be seen, the particles are substantially spherical. FIG. 4B shows a single particle at 300X magnification. The surface is covered with macropores that are greater than 1 micron_in diameter. Many of the pores have a diameter greater than 5 micron with some having diameters greater than 20-30 microns. FiG. 4C shows the surface of the particle at 4000X magnification In a region that does not contain a high number of pores. The single pore shown is about 1 micron in diameter. FIG. 4D shows the central region of the particle of FIG. 2B at 2000x magnification. Surface and interior pores are apparent. FIG. 4E shows a dehydrated PVA particle at 300x magnification that has been mechanically split. Surface and interior pores are apparent. FIG. 4F shows the interior surface of the split particle of FIG. 4E at 2000x magnification. The interior pores are clearly interconnected.

FIG. 5 is a photograph illustrating a pressure reduction device.

FIG. 6 is a photograph depicting a pressure reduction device.

FIG. 7 is a photograph depicting a prepackaged syringe containing embolization particles that have not been subjected to a reduction in pressure.

FIG. 8 is a photograph depicting standard prior art hydration techniques in which particles are hydrated without subjecting the suspension to a reduction in pressure.

FIG. 9 is a photograph depicting embolization particles in suspension in a hydration solution subjected to a reduction In pressure.

FIG. 10 is a photograph illustrating embolization particles suspended in a hydration solution after pressure in the container has been reduced.

FIG. 11 is a photograph showing embolization particles suspended In a hydration solution with a mild inversion mix after a pressure reduction has been applied.

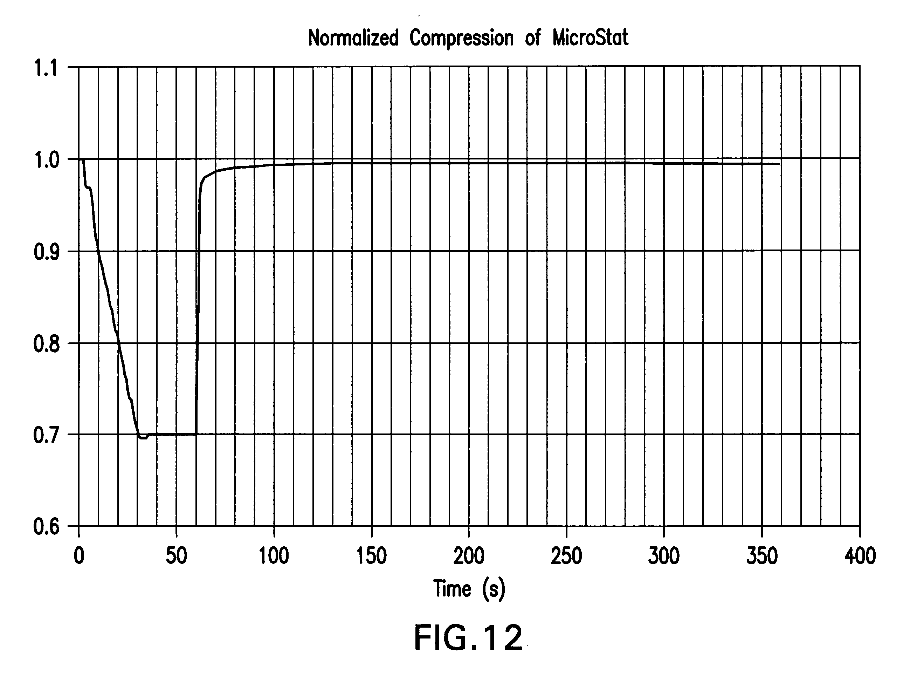

FIG. 12 is a plot of normalized diameter as a function of time for a compression test of a Microstat™ 1000 foam embolization, particle.

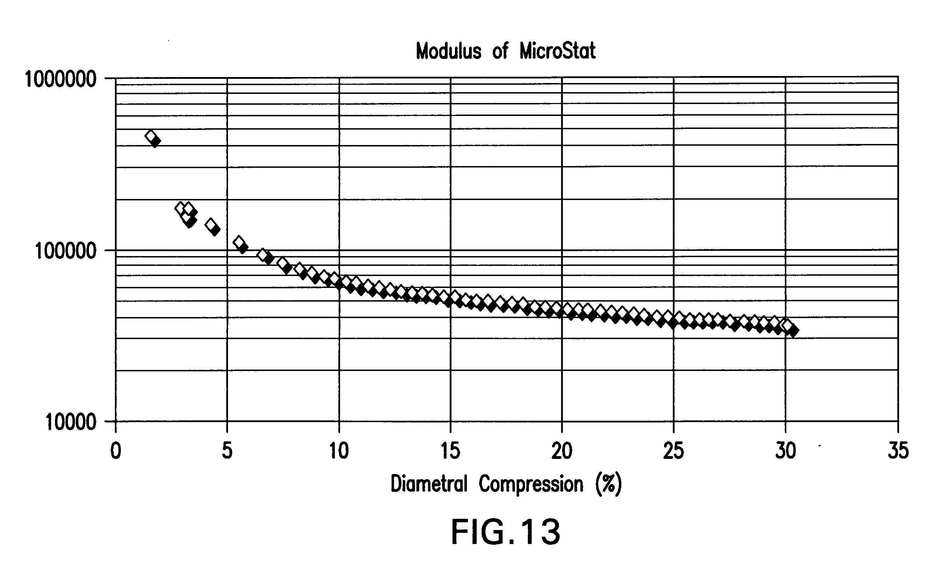

FIG. 13 is a plot of the modulus E as a function of time for a Microsta™ 1000 foam embolization sphere.

The invention is directed to improved substantially spherical porous embolization particles and methods of making them. Methods to rehydrate foam embolization particles including the spherical porous embolization particles are disclosed herein.

## Substantially Spherical Porous Embolization Particles

The substantially spherical porous particles can be used to form artificial embolisms to treat aneurysms, tumors, bleeding, vascular malformations, or otherwise be used to block blood flow to undesired areas by occluding blood vessels, The particle (or a plurality of particles) are preferably made from biocompatible polyvinyl alcohol ("PVA") having interconnected pores that extend from the interior to the surface of the particle.

FIGS. 4A through 4E are scanning electron micrograph (SEM's) of PVA embolization particles made according to the protocols described herein. FIG. 3A shows a population of dehydrated PVA particles. As can be seen, the particles are substantially spherical. FIG. 4B shows a single particle at 300X magnification. The surface is covered with macropores that are greater than 1 micron in diameter. Many of the pores have a diameter greater than 5 microns with some having diameters greater than 20-30 microns.

FIG. 4C shows the central region of the particle of FIG. 2B at 2000x magnification. Surface and interior pores in fluid communication are apparent.

FIG. 4D shows a dehydrated PVA particle at 300x magnification that has been cut. Surface and interior pores are apparent.

FIG. 4E shows the interior surface of the cut particle of FIG. 3D at 2000x magnification. The interior pores are clearly interconnected.

"Substantially interconnected pores" (also referred to as "open") as used in the present application means pores that are in fluid communication with each other within a solid material. According to one embodiment, at least about 20%, 30% 40%, 50 % or 60% of the pores are interconnected. More specifically, larger particles such as 300 and 500 micron dehydrated particles can have at least about 50 % of the pores interconnected. In smaller particles such as 50 micron dehydrated particles, at least about 25 % of the pores of any such small particle of the present invention are interconnected. The interconnected pores define an open, interconnected architecture of volume elements within the particle (see FIGS. 4D, 4E and 4F) that can contain liquid or gas. Some of the internal pores extend to the surface. This provides for more efficient hydration as well as a surface that is more amenable to stable clot formation when used as an embolization particle. In addiction, some portion of the pores within the particles may be closed pores. Generally a minor portion of the pores as determined on a volume to volume basis are closed pores

The term "surface," as used herein, means the exterior portion of the particle. The term "interior portion," as used herein, means any portion of the particle that is interior to the surface.

The particles of the present invention are substantially spherical. The term "substantially spherical" as used herein refers to a generally spherical shape having a maximum diameter/minimum diameter ratio of from about 1.0 to about 2.0, more preferably from about 1.0 to about 1.5 , and most preferably from about 1.0 to about 1.2. This definition is intended to include true spherical shapes and ellipsoidal shapes, along with any other shapes that are encompassed within the maximum diameter/minimum diameter ratio.

According to one embodiment, the pores on the surface of the particle, which are in fluid communication with all or a portion of the pores defined in the interior of the particle, have an average diameter of greater than about 1 micron. Alternatively, the surface pores have an average diameter greater than about 2 or 3 microns.

The particles, in accordance with one aspect of the invention, while compressible to some extent, exhibit a qualitative firmness that is known spherical embolization particles.

The particles of the present invention can be produced in the following manner. First, a PVA solution is produced by mixing polyvinyl alcohol ("PVA") and deionized water and heating the mixture. The amount of PVA added to the mixture ranges from about 7 % to about 22 % by weight of the mixture, preferably about 12 % by weight of the mixture.

A porogen is then added to the mixture. According to one embodiment, the porogen is starch, such as, for example, a mixture of rice starch and deionized water. The amount of starch added to the PVA mixture is the amount that allows the starch to create an effective amount of internal and external pores in the resulting PVA product.

After the porogen is added, a crosslinking agent and crosslinking reactant are added and the resulting product is mixed. For example, according to one embodiment, the crosslinking agent is formaldehyde and the reactant is hydrochloric acid. The solution is de-gassed using a centrifuge to remove air bubbles in the mixture. The de-gassing or centrifuging continues until no bubbles are visible, according to one embodiment. Alternatively, the centrifuging in a four inch radius rotor can be for a period of from about 5 minutes to about 15 minutes.

After de-gassing, the resulting product is added drop-wise to a medium in a preheated reaction vat, which is stirred with a mixing blade. The medium, according to one embodiment, includes a surfactant and a hydrophobic material. The hydrophobic material is included to enhance the formation of spheres. In one aspect, the surfactant is any known surfactant. For example, in one embodiment, the surfactant is Span 80™. The hydrophobic material in one example is mineral oil.

The size of the resulting dehydrated (dry) particles can be influenced by the speed at which the medium is stirred. More specifically, higher revolutions per minute (rpm) generally results in smaller particles. For example, adding the mixture to a medium stirred at 200 rpm produces more 300 to 500 micron particles than any other size. That is, the resulting particles range in size from 45 microns to 1,400 microns, but more particles are produced in the 300 to 500 micron range. Adding the mixture to a medium stirred at 300 rpm produces more particles having diameters less than 300 microns.

The porosity characteristics of the particles are controlled by the production process. During the process, the porogen begins to separate from the PVA prior to PVA crosslinking, in a fashion similar to oil and water separating. As the porogen separates, porogen particles having larger volume within the crosslinking PVA. Thus, the longer the period of time that the mixture has to separate into porogen and PVA prior to crosslinking, the larger the pores. Given that the crosslinking occurs first at the outer portion of the mixture, thereby preventing further separation, the pores at the outer portiuons, particularly the surface, are fewer in number and usually smaller relative to the interior pores.

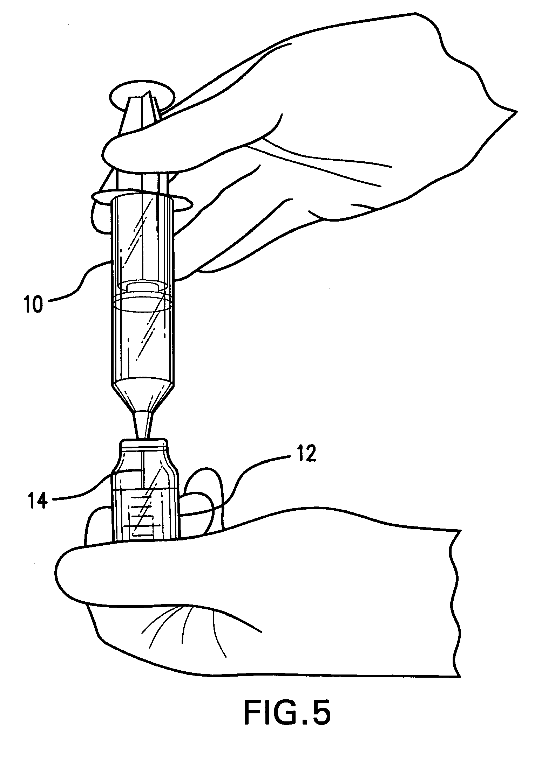

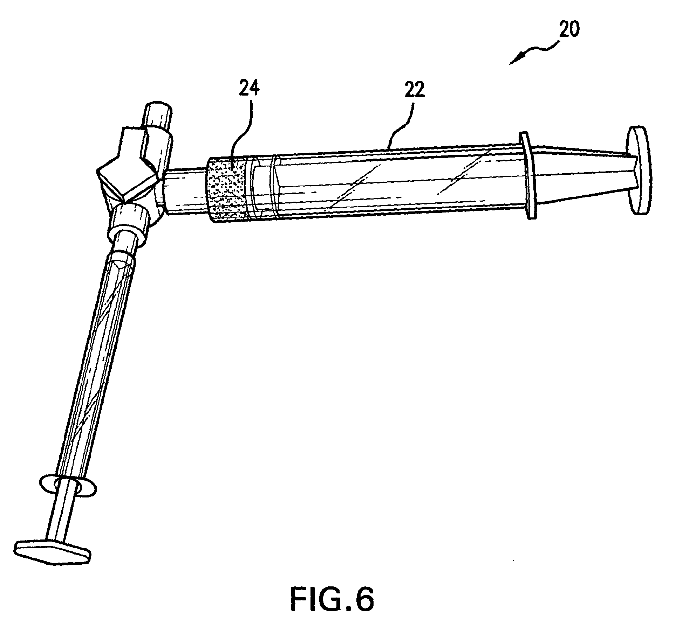

The particles are then dried, size sieved and packaged in a container such as a syringe or vial. The particle container can be combined with at least one of (1) a container of hydration fluid, e.g.. saline with or without contrast agent, (2) a hydration pressure reduction apparatus such as the syringe of FIG. 5 or the apparatus of FIG. 6, which includes two syringes and a 3-way stopcock and (3) instructions for preparation and/or application of the particles to form a kit.

Prior to use, dehydrated particles of the invention are rehydrated. The particles can be quickly hydrated using the hydration and pressure reduction process disclosed herein. The particle(s) can then be positioned in a target region of a blood vessel to occlude the vessel by a delivery system, such as an infusion catheter.

In one aspect of the invention, the apparatus is provided as a kit.

One disadvantage relating to the preparation of dry_PVA particles using known technology is the need to continuously re-mix the suspension of particles to avoid stratification and clumping. Another disadvantage is the risk of unevenly or incompletely hydrated suspensions being injected through a catheter to an embolization target site. Such incompletely-hydrated particles contain residual air pockets in the foam structure which can cause unevenly mixed material and stratification. Such unevenly mixed material and stratification can result in a loss of precise injection control, which can cause such problems as the inadvertent injection of large particle clumps. This can cause a subsequent unintended or overly-large embolization. A further disadvantage of uneven delivery and packing of clumps or stratified particles is that it can cause a catheter blockage, necessitating the emergency removal and replacement of the catheter during the procedure.

Yet another disadvantage of the standard preparation for PVA particles is the possibility of wasted time and expense caused by the length of time required for known hydration technologies. For example, several vials may be used during a typical procedure. Each additional vial means additional time required for hydration. The additional time adds extra expense to the procedure and possible extra radiation exposure to the medical personnel and patient. Further, such time requirements can be life threatening during an emergency situation, such as epistaxis, abdominal bleeding, or postpartum bleeding, when fast delivery of the embolic material is essential.

## TABLE I
### CATHETER COMPATIBILITY OF EMBOSPHERE MICROSPHERES

| From the Manufacturer's Catheter Compatibility Chart | | | Embosphere Microspheres (microns) | | | | | |
|---|---|---|---|---|---|---|---|---|
| Catheter ID (inches) | Catheters | MFG | 40-120 | 100-300 | 300-500 | 500-700 | 700-900 | 900-1200 |
| 0.035-0.038 | All 4Fr and 5F | | X | X | X | X | X | X |
| 0.028 | EmboCath | BioSphere | X | X | X | X | X | |
| 0.024-0.027 | | Terumo | | | | | | |
| | Progreat | Bost Sci | | | | | | |
| | FasTracker-325 | Bost Sci | X | X | X | X | X | |
| | Renegade Hi-Flo | Cordis | | | | | | |
| | MassTransit | Micro | | | | | | |
| | Rebar-027 | Ther | | | | | | |
| 0.019-0.023 | RapidTransit | Cordis | | | | | | |
| | Renegade | Bost Sci | X | X | X | X | | |
| | TurboTracker-18 | Bost Sci | | | | | | |
| 0.014-0.018 | Regatta | Cordis | | | | | | |
| | Prowler-10 | Cordis | X | X | X | | | |
| | Prowler-14 | Cordis | | | | | | |
| | Tracker Excel-14 | Bost Sci | | | | | | |
| 0.008-0.130 | Spinnaker Elite 1.5 | Bost Sci | | | | | | |
| | Spinnaker Elite 1.8 | Bost Sci | X | X | | | | |
| | Magic 1.5 | Balt | | | | | | |

## TABLE II
### COMPRESSIBILITY OF EMBOSPHERE MICROSPHERES

**EMBOSPHERE**

| | High End of Particle Range in Microns | High End of Particle Range in Inches | Embosphere Compatible Smallest ID Size (Inches) | | Compression [1] |
|---|---|---|---|---|---|
| | 300 | 0.011811 | | 0.008 | 32.3% |
| | 500 | 0.019685 | | 0.014 | 28.9% |
| | 700 | 0.027559 | | 0.019 | 31.1% |
| | 900 | 0.035433 | | 0.024 | 32.3% |
| | 1200 | 0.047244 | | 0.035 | 25.9% |

1 Embosphere Catheter Compatibility Chart states up to 33% compression

**TABLE III**

**CATHETER COMPATIBILITY OF CONTOUR SE MICROSPHERES**

| From the Manufacturer's Catheter Compatibility Chart | | | Contour SE Microspheres (microns) | | | | |
|---|---|---|---|---|---|---|---|
| Catheter ID | Including Catheters | MFG | 100-300 | 300-500 | 500-700 | 700-900 | 900-1200 |
| 0.038 | Selective 4Fr and 5F | Bost Sci | X | X | X | X | X |
| 0.024 | FasTracker-325 Renegade Hi-Flo | Bost Sci | X | X | X | X | |
| 0.021 | Renegade-18 | Bost Sci | X | X | X | | |
| 0.013 | Excelisor SL-10 | Bost Sci | X | X | | | |
| 0.011 | Spinnaker Elite 1.5Fr | Bost Sci | X | | | | |

**TABLE IV**

**COMPRESSIBILITY OF CONTOUR SE PARTICLES**

**CONTOUR SE**

| High End of Particle Range in Microns | High End of Particle Range in Inches | Contour SE Compatible Smallest ID Size (Inches) | Compression |
|---|---|---|---|
| 300 | 0.011811 | 0.011 | 6.9% |
| 500 | 0.019685 | 0.013 | 34.0% |
| 700 | 0.027559 | 0.021 | 23.8% |
| 900 | 0.035433 | 0.024 | 32.3% |
| 1200 | 0.047244 | 0.038 | 19.6% |

**TABLE V**

**COMPRESSIBILITY OF SURGICA MICROSTAT™ PARTICLES[1]**

| Dehydrated Particle Size Range | microns Dehydrated Mean Diameter | microns Hydrated Mean Diameter | % Increase | microns Hydrated Maximum Diameter | inches Hydrated Maximum Diameter | inches Minimum ID Delivery Catheter | Calculated % Compression |
|---|---|---|---|---|---|---|---|
| 90-180 μm | 120 | 145 | 21% | 271 | 0.010669 | 0.01 | 6.3% |
| 180-300 μm | 222 | 310 | 40% | 524 | 0.02063 | 0.018 | 12,7% |
| 300-500 μm | 344 | 521 | 51% | 801 | 0.031535 | 0.025 | 20.7% |
| 500-710 μm | 546 | 804 | 47% | 1087 | 0.042795 | 0.035 | 18.2% |
| 710-1,000 μm | 820 | 1205 | 47% | 1437 | 0.056575 | 0.035 | 38,1% |
| 1.000-1.400 μm | 1138 | 1514 | 33% | 1737 | 0.068386 | 0.035 | 48.8% |
| | | mean: | 40% | | | | |

Note: Hydrated in 50% non-ionic contrast and 50% saline

[1] The Microstat particles are substantially spherical porous embolization particles made by Surgica Corporation (El Dorado Hills, California).

**Methods and Systems of Hydration of Intravascular Embolization Particle**

According to one embodiment, the step of rehydrating involves subjecting dehydrated embolization particles to reduced pressure after they have been placed in suspension in a hydration solution. The reduced pressure provides for the removal of residual air bubbles trapped in the foam structures, thereby enhancing hydration and dispersion of the particles in the hydration solution.

It is desirable to quickly and completely hydrate embolization particles and to form a stable homogeneous suspension of particles. Separation of the particles after hydration and mixing, where particles float and/or sink, is undesirable. Ideally, substantially all of the particles should behave the same way in any given hydration fluid. Unfortunately, the use of hydration processes where foam embolization particles are simply mixed with hydration fluid often results in their separation after hydration. The particles hydrate at differing times and exhibit dumping and stratification in the hydration solution. The problem relates to characteristics that vary from particle to particle. Each particle can have a unique configuration with respect to the naturally-occurring air bubbles entrapped in the particle. That is, the variation In the naturally-occurring entrapped air bubbles in each particle can cause bulk density differences across particles, thereby resulting in particles with varying hydration and suspension characteristics such that particles disperse in a non-uniform fashion In the hydration fluid. Thus, the hydration of such particles by simple mixing results is a suspension of clumped or stratified particles that fail to hydrate at the same time and to the same degree.

The methods and apparatuses disclosed herein in contrast, enhance the uniform hydration and dispersion of foam embolization particles in the hydration fluid. According to one embodiment, the reduction of pressure in a container of foam embolization particles and hydration fluid creates substantially uniform density among the particles and results in substantially homogenous suspension characteristics of the particles, thereby enhancing the consistency of the particle suspension and minimizing stratification and clumping.

According to one embodiment, the step of rehydrating includes reducing the pressure in a device containing foam embolization particles after the particles have been suspended In hydration fluid. The hydration fluid is any known fluid for hydrating embolization particles including contrast agent, saline, or any combination thereof, where it is understood that the term "saline" encompasses any salt solution acceptable for physiological use. It is understood that, according to certain embodiments of the present invention, the hydration fluid may contain contrast agent or may be a contrast agent. In one aspect, the pressure reduction is applied immediately after the particles are placed in the fluid. The pressure reduction has a magnitude and duration sufficient to remove residual air bubbles trapped in the foam structures of the particles. The pressure reduction and resulting removal of residual air bubbles can shorten the amount of time required to hydrate the particles and enhance the uniformity of the particles in suspension.

In one aspect of the invention, the pressure reduction is combined with slow agitation such as slowly moving the container of embolization particles back and forth between an upright position and an inverted position. Alternatively, the agitation is vigorous agitation caused by shaking the container. In a further alternative, the agitation can be any level of agitation that provides for a desirable suspension and hydration of the particles.

In one embodiment, the pressure in a container containing the particles is reduced more than once. For example, the particles can be subjected to reduced pressure in repetitive cycles. According to this embodiment, a first pressure reduction is applied to the particles and then a second pressure reduction is applied. Alternatively, pressure is reduced in a repetitive series ranging from about 2 applications to about 5 applications. Such repeated applications of pressure reduction can be more effective than one application, depending on the porosity and other characteristics of the particles.

The apparatus used to reduce pressure in a container of embolization, particles and hydration fluid is a syringe. In one aspect of the invention, the apparatus is the syringe that is also used as part of a delivery device for application of the particles to the target vessel. FIG. 5 depicts a 20 ml syringe 10 configured to reduce the pressure of embolization, particles in hydration fluid. The needle 14 of the syringe is inserted into a vial 12 containing dry foam embolization particles. The vial 12 is a prepackaged vial of embolization particles.

Hydration fluid is added to the vial typically by a syringe. The vial is preferably vented to bring the pressure in the vial to ambient conditions prior to pressure reduction. The needle 14 of syringe 10 is positioned in the vial 12 such that the needle opening is in fluid communication with the gas in the vial rather than the fluid, and then the plunger 14 is withdrawn by a predetermined amount, thereby reducing pressure for a predetermined period of time. This results in degassing of the particles which facilitates hydration. In the embodiment depicted in FIG. 5, the vial has a volume of 10 ml plus the additional "head space" volume in the vial, which is the space in the collar portion of the vial, and the amount of fluid present in the vial is about 10 ml. The plunger 14 is withdrawn to the 10 ml mark for 15 seconds. Alternatively, the plunger 14 can be withdrawn by any predetermined amount for any predetermined amount of time that enhances hydration and uniform suspension of the particles. According to this embodiment, where larger syringes are used to create a greater pressure reduction, the syringe plunger displacement to remove gas from the 10 ml vial ranges from about 2 ml to about 60 ml, more preferably from about 4 ml to about 40 ml, and most preferably from about 8 ml to about 15 ml.

At these pressures, the duration of the pressure reduction ranges from about 5 seconds to about 30 seconds, more preferably from about 10 seconds to about 20 seconds, and most preferably about 15 seconds. However, it is understood by those skilled in the art that a longer duration will achieve the same effect.

FIG. 6 depicts an alternative pressure reduction apparatus 20, according to another embodiment of the present invention. The apparatus 20 is an embolization procedure apparatus having a 20 ml syringe 22 containing approximately 100 mg of dry crosslinked PVA foam embolization particles 24, and an empty 10 ml syringe 25, both syringes being connected to a connection component 26. In the embodiment depicted in FIG. 6. the connection component 26 is a three-way stopcock having a connection hub 28. Alternatively, the syringe 22 can contain any useful amount of foam em bolization particles 24.

The apparatus of FIG. 6 is used in the following manner. First, according to one embodiment, hydration fluid is added to syringe 22. The 20 ml syringe 22 is uncoupled from the apparatus 20 and loaded with hydration fluid. For example, 10 ml of hydration fluid is loaded into the syringe 22. Alternatively, instead of uncoupling the syringe from the apparatus 20, the stopcock 26 is adjusted to allow for fluid communication between the connection hub 28 and the syringe 22, and the hydration fluid is loaded through the connection hub 28 into the syringe 22. The stopcock 26 is then adjusted to seal the syringe 22 and the plunger of syringe 22 is withdrawn 10 ml and held for 15 seconds, thereby reducing pressure within the syringe 22. Alternatively, the plunger is withdrawn an amount ranging from about 5 ml to about 15 ml. In a further alternative, the plunger is held in the withdrawn position for a period ranging from about 10 seconds to about 30 seconds. In one aspect of the invention, the plunger can be held in the withdrawn position using a VacLok™ syringe, available from Merit Medical (South Jordon, Utah).

The plunger of syringe 22 is then released. Typically, the pressure reduction causes gas to be removed from the pores of the embolic particles and thus causes the gas to collect as microbubbles visible in the hydration fluid. The stopcock is then adjusted to create fluid communication between syringe 22 and syringe 25. Once they are in communication, a transfer step is performed in which the plunger of syringe 22 is depressed, thereby transferring the particles and fluid into syringe 25. The transfer step enhances hydration and dispersion of the particles, helps to disassociate the microbubbles from the particles and facilitates the coalescence of the microbubbles present in the fluid. According to one embodiment, the transfer step is performed again, with the plunger of syringe 25 being depressed, thereby transferring the fluid and particles back to syringe 22. This transfer step can be repeated several times, for example, from about 1 time to about 20 times.

The fluid and particles, which are returned to syringe 22 after the transfer step, are again subjected to a pressure reduction as necessary. It is apparent that hydration and or particle dispersion is not yet complete, the plunger of syringe 22 is withdrawn 10 ml for 15 seconds. Alternatively, the plunger can be withdrawn by any reasonable amount for any reasonable amount of time. The syringe can also be subjected to agitation at this point, thereby further enhancing hydration, disassociation of the microbubbles from the particles, and coalescence of the microbubbles.

When the microbubbles have coalesced and separated from the hydration fluid, the gas present in the syringe 22 can be discharged from the syringe. That is, the syringe 22 is positioned so that the gas pocket is at the end of the syringe 22 connected to the stopcock 26 and then the stopcock 26 is adjusted to create fluid communication between the syringe 22 and the surrounding environment. Once fluid communication is established, the plunger is depressed to vent the gas out through the stopcock 26.

In one aspect of the invention, after venting, the stopcock 26 can be adjusted to again create fluid communication between the syringes 22 and 25 and once again perform the transfer step as many times as need to successfully hydrate and disperse the particles within the suspension.

Once the desired suspension is achieved with the particle suspension contained in the syringe 25, the connection hub 28 is connected to the catheter (not shown) for delivery of the particles to the patient. The stopcock 26 is adjusted to create fluid communication between the syringe 25 and the hub 28 and the plunger of syringe 25 is depressed to inject the particles into the catheter. Alternatively, the particles can be contained in and injected from syringe 22.

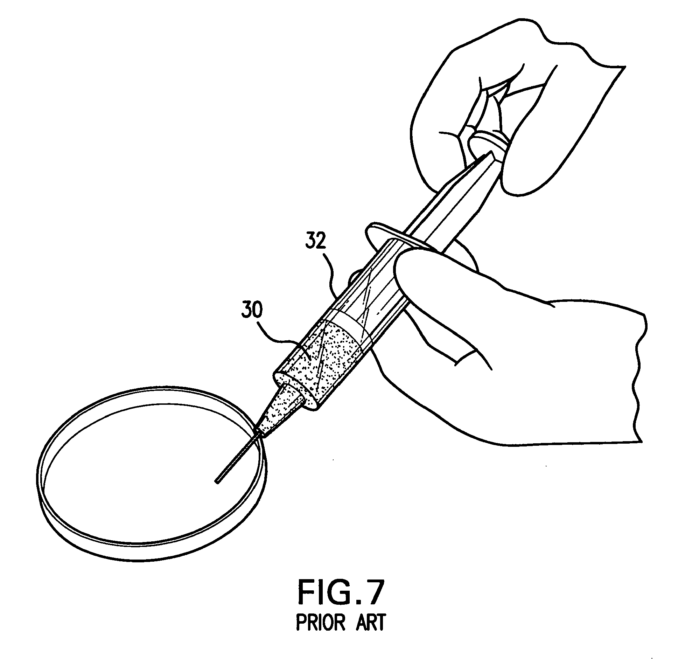

If no pressure reduction is applied, the particles exhibit stratification and clumping in the top and bottom portions of the syringe. (See FIG. 7) The floating particles represent incompletely hydrated particles.

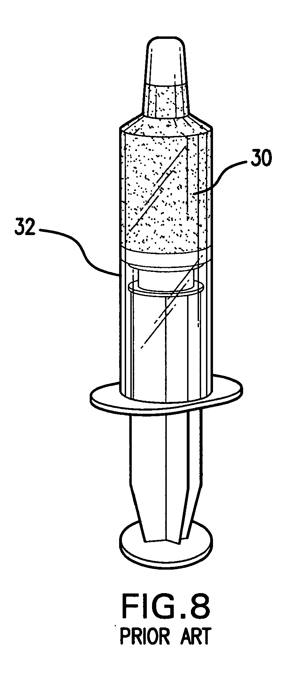

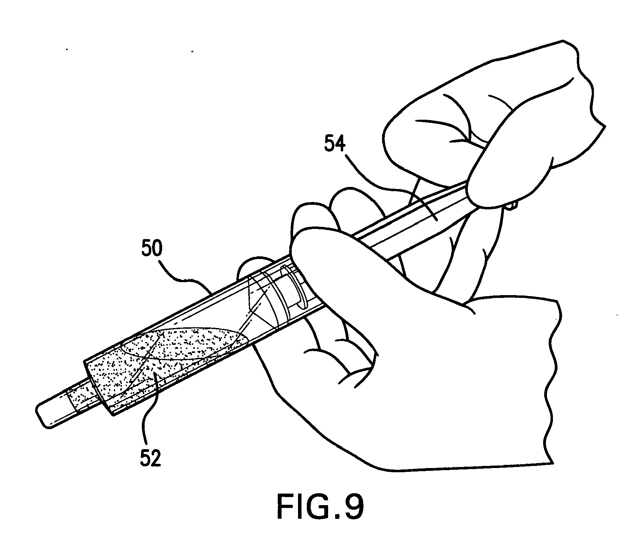

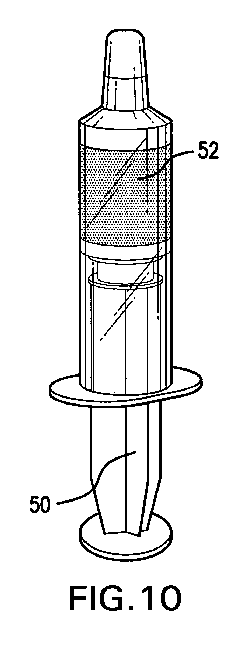

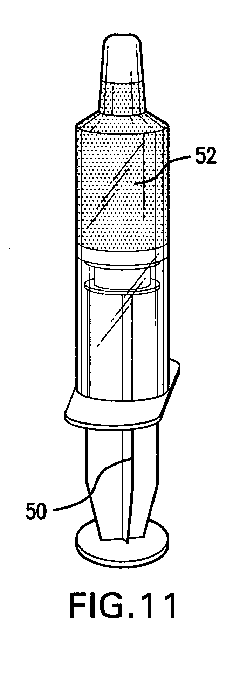

In contrast, the use of reduced pressure reduces stratification and clumping. For examples, a prepackaged 20 ml syringe containing crosslinked PVA foam embolization particles 52 after hydration with 10 ml of saline and during pressure reduction created by withdrawing the plunger 54 by an amount equal to 10 ml for 15 seconds. FIG. 10 depicts the syringe 50 of FIG. 9 immediately after the pressure reduction pull. The particles 52 are dispersed uniformly near the bottom of the syringe 50 in comparison to the stratified particles 30 depicted in FIG. 8. Further, FIG. 11 depicts the particles 52 of FIGS. 9 and 10 after the syringe has been agitated with a mild inversion agitation in which the syringe was repeatedly and mildly moved from an upright position to an inverted position. As can be seen in the figure, the particles 52 are dispersed uniformly throughout the syringe 50.

The pressure reduction apparatus can be any known device capable of reducing the pressure on embolization particles and hydration fluid in a container, including, for example, any commercially available vacuum pump capable of reducing pressure in a container, including a vial or syringe. The apparatus can be used independently or in conjunction with an embolization particle delivery device.

In one aspect of the invention, a kit is provided. The kit, according to one embodiment, contains a suitable amount of embolization particles and a hydration/pressure reduction apparatus such as the syringe of FIG. 5 or the apparatus of FIG. 6, which includes two syringes and a stopcock. Alternatively, the kit can also include an appropriate hydration solution, a syringe or other suitable particle delivery device, and, according to one embodiment, instructions for preparation and application of the particles. The particles can be in the syringe or in a vial sealed with a septum.

Although the disclosure herein refers to particles, the use of single particles is also contemplated.

## EXAMPLES

### Example 1

*Production of Embolization Particles.*

A mixture of 24.0 grams of polyvinyl alcohol and 176.0 grams of deionized water was rapidly heated to 100° C and held for 12 minutes. Then 150.6 grams of the material was transferred for reaction purposes and set aside and allowed to cool. A separate mixture of 1.5 grams of rice starch and 135 grams of deionized water was heated to 80° C and then 48.4 grams of the material was added to the PVA solution and thoroughly mixed. To this mixture 21.7 grams of concentrated hydrochloric acid and 22.0 grams of about 37% formaldehyde (formalin solution) were added to form the reaction PVA mixture. The mixture was then centrifuged at a fast speed (but not so fast that the PVA solution and starch are caused to separate), which, according to one embodiment, is 2,000 rpm with a centrifuge having a 4-inch radius for 8 minutes to remove microbubbles of air trapped in the mixture. The mixture is then added drop-wise to a reactant medium made up of 160 grams Span 80™ and 3,840 grams USP mineral oil stirring at 200 RPM in a reaction vat and preheated to 55° C. Once all the reaction PVA mixture is added, the set point of the reaction PVA mixture is set to 30° C and allowed to react for 16 hours.

Once reacted, the resultant crosslinked PVA spheres were cleaned of mineral oil, Span 80™, hydrochloric acid, formaldehyde, and starch. The cleaning process involved dumping the resultant spheres from the 16-hour reaction over a 45-micron screen such that the beads were trapped on the screen while some of the reactants passed through. Then, chloroform was poured over the top of the beads and through the screen, thereby washing off more of the unwanted materials. Further, the beads were then washed with Triton-X 100™ using a standard ultrasonic cleaning machine. Subsequently, the success of the washing process was determined by testing the particles were tested for any remaining, unwanted reactants. The particles were then dried in an oven and separated into size ranges using ASTM standard sieves.

### Example 2

In this example, embolization particles were made using a slightly different process. The same procedure as in Example 1 was followed, but the rpm of the PVA reaction medium was increased to 300 rpm. The resulting particles exhibited the same mechanical properties as the spheres in Example 1, but the average size of these particles was smaller than the average size of the Example 1 particles. Without being limited by theory, it is believed based on these results that the rpm of the PVA reaction medium can be manipulated to obtain various desired particle sizes.

### Example 3

In this example, embolization particles were made using a slightly different manufacturing process. The same procedure as in Example 1 was followed, except that the mass of the PVA used was 33.52 grams to make a 16% PVA solution (as opposed to a 12% solution in Example 1). The resulting particles exhibited Increased firmness and resilience.

### Example 4

In this example, embolization particles were made using a slightly different manufacturing process. The same procedure as in Example 1 was followed, except that the mass of formalin solution was increased to 33 grams. The resulting particles exhibited slower hydration than particles produced in the previous examples, but eventually sank in the water. Without being limited by theory, it is believed that the greater concentration of formalin caused a greater extent of conversion of the PVA to the acetal functionality. The rate and extent of the acetal formation reaction is known to be directly related to temperature and acid and formaldehyde concentration.

### Example 5

A TA Instruments TMA Q400EM Thermomechanical Analyzer equipped with a standard Expansion Probe Assembly (TA instruments Part Number 944122.901) was used to perform a multi-sequence compression test on each microsphere. The instrument was calibrated according to the manufacturer's instructions prior to the start of the tests. A TA instruments aluminum differential scanning calorimetry ("DSC") pan (TA Instruments Part Number 900786.901), filled with a small amount of hydration fluid, was used to hold each microsphere during the compression testing sequences. Prior to each test, however, the TMA's probe ("the probe") displacement was zeroed using each respective microsphere's dry DSC pan to compensate for the thickness of the DSC pan. In doing so, the indicated displacement of the DSC probe corresponded to the measured diameter of the microsphere.

The microspheres were prepared according to the manufacturer's directions for use in a 50 percent by volume solution of contrast agent (Optiray 320®, Tyco Healthcare) in saline. An individual microsphere was taken from the hydration fluid and placed into a DSC pan, along with a small amount of hydration fluid. The DSC pan containing the microsphere was positioned underneath the probe such that the centerline axis of the microsphere visually corresponded with the centerline axis of the probe. The probe was lowered by hand onto the top of the microsphere to measure the diameter of the microsphere. The downward force exerted by the probe was 0.002 N during the measurement. The initial diameter of the microsphere was entered into the instrument control software. The TMA's furnace was lowered over the microsphere and the compression testing sequence was begun. First, the temperature was allowed to equilibrate to 23.0 ± 0.1 degrees Celsius. Second, the probe was commanded to ramp the compressive strain from 0.00 percent to 30.00 percent at a rate of 60.00 percent strain per minute. Third, the probe was commanded to hold the compressive strain at 30.00 percent for 30 seconds. Fourth, the probe force was reduced to 0.002 Newtons for 5 minutes. During each sequence of the compression test, data (time in minutes, temperature in degrees Celsius, dimension change in micrometers, force in Newtons, strain in percent, sample length in millimeters, and derivative of sample length in millimeters per minute) were recorded at 0.5 second Intervals. The resulting ASCII text files were imported into a Microsoft Excel template for subsequent analyses. The Microsoft Excel template generated a plot of normalized diameter (initial diameter = 1.00) as a function of time (see Fig. 1). In addition, the Excel template generated a plot of modulus, E, as a function of compressive strain (see FIG. 1) using the equation mentioned by Hertz (1978) and developed by Timoshenko et al. (1970) for a sphere compressed between two parallel plates:

$$E = 0.75F(1 - \upsilon^2)\frac{1}{D^{3/2}}\frac{1}{R^{1/2}} \quad ,$$

where D was the deformation of the microsphere, R was the radius of the microsphere, F was the applied, force, and □ was Poisson's ratio (assumed to be 0.5 for this work). For each microsphere product size, six replicate tests were performed on one lot number.

Table 1. Summary of Compression Data

| Manufacturer | Surgica | BioCure, Inc. | Boston Scientific | Biosphere Medical |
|---|---|---|---|---|
| Product | MicroStat™ | BeadBlock™ | Contour SE™ | Embosphere® |
| Size, μm | 1000 | 500-700 | 900-1200 | 900-1200 |
| Lot Number | E06009 | 50802-6 | 8181216 | 178GB5 |
| Diameter by TMA, μm | 1728 ± 209 | | | |
| Normalized Diameter After 2 sec of Recovery (%) | 95.0 ± 1.4 | N/A | N/A | N/A |
| Normalized Diameter After 60 sec of Recovery (%) | 99.3 ± 0.1 | N/A | N/A | N/A |
| Modulus at 10 ± 0.5% Compression (kPa) | 76.9 ± 7.2 | N/A | N/A | N/A |
| Modulus at 30 ± 0.5% Compression (kPa) | 39.7 ± 6.0 | N/A | N/A | N/A |
| N/A - data not available due to the inability of the particles to maintain initial diameter under the 0.002N resting force. | | | | |

*Example 6*

*SEM Images*

MicroStat samples were stored dry in sealed containers. They were hydrated in saline. Embosphere and Contour SE were purchased samples sold hydrated in saline in sealed containers. It was not possible to image the hydrated samples of Embosphere or Contour SE as they collapsed in even a low vacuum environment (environmental scanning electron microscopy).

The instrument used was a Quanta 600 Environmental Scanning Electron Microscope manufactured by the FEI Company (Hillsboro, Oregon) and available at the Scripps Institute of Oceanography (San Diego, CA).

All samples were prepared (methods well known in the art) by Critical Point Drying, exchanging 200 proof ethanol for the saline hydration fluid, then exchanging the ethanol with liquid CO2 (4x), then finally heating to drive the $CO_2$ off as a gas. The samples were then transferred to carbon tape and sputter coated with gold/palladium. The samples were then imaged under high vacuum SEM.

The SEM's for Embosphere™, Contour SE™, Bead Block™ and the Surgica Microstat™ spherical porous embolization particles are set forth in FIGS. 1-4, respectively.

*Example 7*

In the instant example, particle hydration was compared for two different embolization particle suspensions in their original 10 ml containers in which one suspension was subjected to a reduction in pressure while the other suspension was not.

*Materials*

Prepackaged PVA foam embolization particles were hydrated in their original sterile 10 ml serum type glass vial container. The container contained approximately 100 mg of dry PVA foam particles. Ten (10) ml of 50% contrast agent diluted with saline was injected into the 10 ml vial using a syringe fitted with a standard 21 gage needle. The contrast agent was Oxilan™ 300.

*Methods*

The control suspension was not subjected to a reduction in pressure.

The test suspension was subjected to a pressure reduction. After total injection of the hydration solution and release of the pressure thus created in the vial, the needle was positioned in the air space above the fluid mixture, and the empty syringe plunger was withdrawn by 10 ml, which produced a reduction in pressure in the container. After 15 seconds, the pressure reduction was released by allowing the plunger to move back to a position of rest.

*Results*

With respect to the test suspension in which the pressure was reduced, the particles were observed to be rapidly and evenly hydrated in the fluid within the vial. In contrast, a normal hydration time, without the aid of the pressure reduction technique, showed incomplete hydration after 10 minutes, and even after more mixing, a significant percentage of the particles were still not fully hydrated.

*Example 8*

In this example, particle hydration was compared for two different embolization particle suspensions hydrated in their original 20 ml syringe containers in which one suspension was subjected to a pressure reduction while the other suspension was not.

*Materials*

Prepackaged PVA foam embolization particles were hydrated in their original 20 ml syringe container. The syringe contained approximately 100 mg of dry PVA foam particles. Ten (10) ml of 50% contrast agent diluted with saline was aspirated into the syringe. The contrast agent was Oxilan™ 300.

*Methods*

The control suspension was not subjected to a reduction in pressure.

The test suspension was subjected to a reduction in pressure. After aspiration of the contrast agent diluted with saline into the syringe (at which point the contents of the syringe are at ambient pressure), the syringe was fitted with a luer connector cap and the cap was tightened to create an air tight seal. The syringe plunger was then withdrawn an additional 10 ml, which produced a reduction in pressure with respect to the mixture. After 15 seconds, the pressure reduction was released by allowing the plunger to move back to a position of rest.

### Results

With respect to the test suspension in which the pressure was reduced, the particles were observed to be rapidly and evenly hydrated in the fluid within the syringe. In contrast, a normal hydration time, without the aid of the pressure reduction technique, showed incomplete hydration after 10 minutes, and even after more mixing, a significant percentage of the particles were still not fully hydrated.

### Example 9

In this example, particle hydration was compared for two different embolization particle suspensions hydrated in prepackaged 20 ml locking syringe containers in which one suspension was subjected to a pressure reduction while the other suspension was not.

### Materials

An embolization procedure kit containing, as a component, a prepackaged 20 ml locking syringe container with approximately 100 mg of dry PVA foam embolization particles, was hydrated with a normal saline solution. Ten (10) ml of saline was aspirated into the locking syringe.

### Methods

The control suspension was not subjected to a reduction in pressure.
The test suspension was subjected to a pressure reduction. After aspiration of the saline solution into the locking syringe, the syringe was fitted with a luer connector cap and the cap was tightened to create an air tight seal. The syringe plunger was then withdrawn an additional 90 ml which resulted in a reduction in pressure. The syringe plunger was locked into position at the 20 ml mark. After 15 seconds, the reduced pressure was released by allowing the plunger to move back to a position of rest.

### Results

With respect to the test suspension in which the pressure was reduced, the particles were observed to be rapidly and evenly hydrated in the fluid within the syringe. in contrast, a normal hydration time, without the aid of the pressure reduction technique, showed incomplete hydration after 10 minutes, and even after more mixing, a significant percentage of the particles were still not fully hydrated.

### Claims

1.   A dehydrated substantially spherical embolization particle for use in a method of embolization,
wherein the particle comprises a crosslinked polymer, wherein the interior of said particle comprises interconnected pores that extend to the surface of the particle, wherein at least 20% of the pores are interconnected, wherein the surface pores have an average diameter of greater than 1 $\mu$m (micron), and wherein the particle has a diameter ranging from 45 to 1400 $\mu$m (microns); and
wherein the method of embolization comprises the steps of rehydrating the dehydrated particle, and positioning the rehydrated particle in a target region of a blood vessel.

2.   The particle of claim 1, wherein the surface pores have an average diameter greater than 2 $\mu$m (microns); or wherein the particle has a diameter ranging from 100 to 700 $\mu$m (microns).

3.   The particle of claim 1 or 2, wherein at least 30% of the pores are interconnected.

4.   The particle of any of claims 1 to 3, wherein the polymer comprises polyvinyl alcohol.

5.   The particle of claim 4, wherein the surface pores have a diameter greater than 5 $\mu$m (microns).

6.   The particle of any of claims 1 to 5, wherein the step of rehydrating the dehydrated particle comprises placing the dehydrated particle in a suspension of a hydration fluid and subjecting the particle to reduced pressure.

7. The particle of claim 6, wherein the hydration fluid is a contrast agent, saline or any combination thereof.

8. The particle of claim 6 or 7, wherein the pressure is reduced such that air bubbles in the particles are removed; and/or wherein the pressure is reduced more than once; and/or wherein the pressure is reduced using a syringe or vacuum pump.

9. An embolization device comprising (i) a catheter, and (ii) the embolization particle of any of claims 1 to 8.

10. A container comprising the particle of claim 4 or 5.

11. The container of claim 10, wherein the container is a syringe or vial.

12. A kit comprising the container of claim 10 or 11, and further comprising at least one of (i) a container of hydration fluid, (ii) an apparatus for reducing pressure during hydration, and (iii) instructions for preparation and/or application of the particles.

13. The particle of any of claims 1 to 8 for use in occlusion of blood vessels by embolization.

14. The particle of any of claims 1 to 8 for use in the treatment of aneurysms, tumors, bleeding, or vascular malformations by embolization.

15. A method of producing the particle of claim 4 or 5, comprising:

    (a) mixing polyvinyl alcohol and deionized water, and heating;
    (b) adding porogen to the mixture of (a),
    (c) adding a crosslinking agent and a crosslinking reactant to the mixture of (b), and mixing;
    (d) degassing the mixture of (c);
    (e) adding the degassed mixture of (d) drop-wise to a medium comprising a surfactant and a hydrophobic material;
    (f) stirring the medium to obtain substantially spherical particles; and
    (g) dehydrating the substantially spherical particles.

**Patentansprüche**

1. Dehydriertes, im Wesentlichen sphärisches Embolisationsteilchen zur Verwendung in einem Embolisationsverfahren, wobei das Teilchen ein vernetztes Polymer umfasst, wobei das Innere des Teilchens miteinander verbundene Poren umfasst, die sich bis zur Oberfläche des Teilchens erstrecken, wobei mindestens 20% der Poren miteinander verbunden sind, wobei die Oberflächenporen einen durchschnittlichen Durchmesser größer als 1 $\mu$m (Mikron) haben, und wobei das Teilchen einen Durchmesser von 45 bis 1400 $\mu$m (Mikron) hat; und wobei das Embolisationsverfahren die Schritte Rehydrieren des dehydrierten Teilchens und Positionieren des rehydrierten Teilchens in einem Zielbereich eines Blutgefäßes umfasst.

2. Teilchen nach Anspruch 1, wobei die Oberflächenporen einen durchschnittlichen Durchmesser größer als 2 $\mu$m (Mikron) haben; oder wobei das Teilchen einen Durchmesser von 100 bis 700 $\mu$m (Mikron) hat.

3. Teilchen nach Anspruch 1 oder 2, wobei mindestens 30% der Poren miteinander verbunden sind.

4. Teilchen nach einem der Ansprüche 1 bis 3, wobei das Polymer Polyvinylalkohol umfasst.

5. Teilchen nach Anspruch 4, wobei die Oberflächenporen einen Durchmesser größer als 5 $\mu$m (Mikron) haben.

6. Teilchen nach einem der Ansprüche 1 bis 5, wobei der Schritt des Rehydrierens des dehydrierten Teilchens das Positionieren des dehydrierten Teilchens in einer Suspension einer Hydrierungsflüssigkeit und das Aussetzen des Teilchens einem reduzierten Druck umfasst.

7. Teilchen nach Anspruch 6, wobei die Hydrierungsflüssigkeit ein Kontrastmittel, eine Salzlösung oder eine Kombination davon ist.

8. Teilchen nach Anspruch 6 oder 7, wobei der Druck so reduziert wird, dass Luftblasen in den Teilchen entfernt werden; und/oder
wobei der Druck mehr als einmal reduziert wird; und/oder
wobei der Druck unter Verwendung einer Spritze oder einer Vakuumpumpe reduziert wird.

9. Embolisationsvorrichtung, die (i) einen Katheter, und (ii) das Embolisationsteilchen nach einem der Ansprüche 1 bis 8 umfasst.

10. Behälter, der das Teilchen nach Anspruch 4 oder 5 umfasst.

11. Behälter nach Anspruch 10, wobei der Behälter eine Spritze oder eine Ampulle ist.

12. Kit, der einen Behälter nach Anspruch 10 oder 11 umfasst, und ferner mindestens eines von (i) einem Behälter mit Hydrierungsflüssigkeit, (ii) einer Vorrichtung zur Druckminderung während der Hydrierung, und (iii) Anweisungen zur Vorbereitung und/oder Anwendung der Teilchen umfasst.

13. Teilchen nach einem der Ansprüche 1 bis 8 zur Verwendung beim Verschließen von Blutgefäßen durch Embolisation.

14. Teilchen nach einem der Ansprüche 1 bis 8 zur Verwendung bei der Behandlung von Aneurysmen, Tumoren, Blutung oder Gefäßmissbildungen durch Embolisation.

15. Verfahren zur Herstellung des Teilchens nach Anspruch 4 oder 5, umfassend:

   (a) Mischen von Polyvinylalkohol und entionisiertem Wasser, und Erwärmen;
   (b) Hinzufügen von Porogen zu dem Gemisch aus (a),
   (c) Hinzufügen eines Vernetzungsmittels und eines Vernetzungsreaktants zu dem Gemisch aus (b), und Mischen;
   (d) Entgasen des Gemischs aus (c);
   (e) tropfenweises Hinzufügen des entgasten Gemischs aus (d) zu einem Medium umfassend einen grenzflächenaktiven Stoff und ein hydrophobes Material;
   (f) Rühren des Mediums, um im Wesentlichen sphärische Teilchen zu erlangen; und
   (g) Dehydrieren der im Wesentlichen sphärischen Teilchen.

## Revendications

1. Une particule d'embolisation essentiellement sphérique déshydratée pour utilisation dans un procédé d'embolisation, où la particule comprend un polymère réticulé, où l'intérieur de la particule comprend des pores interconnectés qui s'étendent jusqu'à la surface de la particule, où au moins 20 % des pores sont interconnectés, où les pores de surface ont un diamètre moyen supérieur à 1 $\mu$m (micron) et où la particule a un diamètre allant de 45 à 1400 $\mu$m (microns); et
où le procédé d'embolisation comprend les étapes de réhydratation de la particule déshydratée et positionnement de la particule réhydratée dans une région cible d'un vaisseau sanguin.

2. La particule de la revendication 1, où les pores de surface ont un diamètre moyen supérieur à 2 $\mu$m (microns); ou où la particule a un diamètre allant de 100 à 700 $\mu$m (microns).

3. La particule de la revendication 1 ou 2, où au moins 30 % des pores sont interconnectés.

4. La particule de l'une quelconque des revendications 1 à 3, où le polymère comprend un alcool polyvinylique.

5. La particule de la revendication 4, où les pores de surface ont un diamètre supérieur à 5 $\mu$m (microns).

6. La particule de l'une quelconque des revendications 1 à 5, où l'étape de réhydratation de la particule déshydratée comprend la mise en place de la particule déshydratée dans une suspension d'un fluide d'hydratation et la soumission de la particule à une pression réduite.

**7.** La particule de la revendication 6, où le fluide d'hydratation est un agent de contraste, solution physiologique ou une combinaison de ceux-ci.

**8.** La particule de la revendication 6 ou 7, où la pression est réduite de manière que des bulles d'air dans les particules sont enlevées; et/ou
où la pression est réduite plus qu'une fois; et/ou
où la pression est réduite en utilisant une seringue ou une pompe à vide.

**9.** Un dispositif d'embolisation comprenant (i) un cathéter, et (ii) la particule d'embolisation de l'une quelconque des revendications 1 à 8.

**10.** Un conteneur comprenant la particule de la revendication 4 ou 5.

**11.** Le conteneur de la revendication 10, où le conteneur est une seringue ou un flacon.

**12.** Un kit comprenant le conteneur de la revendication 10 ou 11 et comprenant en outre au moins un de (i) un conteneur de fluide d'hydratation, (ii) un appareil pour réduire la pression pendant l'hydratation et (iii) des instructions pour préparer et/ou appliquer les particules.

**13.** La particule de l'une quelconque des revendications 1 à 8 pour utilisation dans l'occlusion de vaisseaux sanguins par embolisation.

**14.** La particule de l'une quelconque des revendications 1 à 8 pour utilisation dans le traitement d'anévrismes, tumeurs, saignement ou malformations vasculaires par embolisation.

**15.** Un procédé de production de la particule de la revendication 4 ou 5, comprenant:

(a) mélanger alcool polyvinylique et eau déionisée et chauffer;
(b) ajouter porogène au mélange de (a),
(c) ajouter un agent de réticulation et un réactif de réticulation au mélange de (b) et mélanger;
(d) dégazer le mélange de (c);
(e) ajouter le mélange dégazé de (d) goutte par goutte à un médium comprenant un surfactant et un matériau hydrophobique;
(f) agiter le médium afin d'obtenir des particules essentiellement sphériques; et
(g) déshydrater les particules essentiellement sphériques.

| HFW | Mag | Sig | WD | Spot | HV | 1 mm |
|---|---|---|---|---|---|---|
| 5.12 mm | 50x | SE | 19.52 mm | 3.0 | 20.0 kV | SIO Analytical Facility |

## FIG.1a

| HFW | Mag | Sig | WD | Spot | HV | 100 μm |
|---|---|---|---|---|---|---|
| 0.51 mm | 500x | SE | 19.42 mm | 3.0 | 20.0 kV | SIO Analytical Facility |

## FIG.1b

| HFW | Mag | Sig | WD | Spot | HV | 1 µm |
|---|---|---|---|---|---|---|
| 6.40 µm | 40000x | SE | 19.45 mm | 3.0 | 20.0 kV | SIO Analytical Facility |

## FIG.1c

| HFW | Mag | Sig | WD | Spot | HV | 100 µm |
|---|---|---|---|---|---|---|
| 0.43 mm | 600x | SE | 12.48 mm | 3.0 | 20.0 kV | SIO Analytical Facility |

## FIG.1d

| HFW | Mag | Sig | WD | Spot | HV | 20 μm |
|---|---|---|---|---|---|---|
| 0.13 mm | 2000x | SE | 12.42 mm | 3.0 | 20.0 kV | SIO Analytical Facility |

FIG.1e

| HFW | Mag | Sig | WD | Spot | HV | 1 μm |
|---|---|---|---|---|---|---|
| 6.40 mm | 40000x | SE | 12.44 mm | 3.0 | 20.0 kV | SIO Analytical Facility |

FIG.1f

| HFW | Mag | Sig | WD | Spot | HV | 1 mm |
|---------|------|-----|----------|------|---------|------------------------|
| 5.12 mm | 50x | SE | 19.17 mm | 3.0 | 20.0 kV | SIO Analytical Facility |

## FIG.2a

| HFW | Mag | Sig | WD | Spot | HV | 100 μm |
|---------|------|-----|----------|------|---------|------------------------|
| 0.51 mm | 500x | SE | 19.58 mm | 3.0 | 10.0 kV | SIO Analytical Facility |

## FIG.2b

| HFW | Mag | Sig | WD | Spot | HV | 100 µm |
|---|---|---|---|---|---|---|
| 0.64 mm | 400x | SE | 59.43 mm | 3.0 | 20.0 kV | SIO Analytical Facility |

## FIG.2c

| HFW | Mag | Sig | WD | Spot | HV | 20 µm |
|---|---|---|---|---|---|---|
| 0.13 mm | 2000x | SE | 59.43 mm | 3.0 | 20.0 kV | SIO Analytical Facility |

## FIG.2d

| HFW | Mag | Sig | WD | Spot | HV | 2 μm |
|------|--------|-----|----------|------|---------|------|
| 12.80 μm | 20000x | SE | 19.59 mm | 3.0 | 10.0 kV | SIO Analytical Facility |

FIG.2e

| HFW | Mag | Sig | WD | Spot | HV | 1 μm |
|---------|--------|-----|----------|------|---------|------|
| 6.40 μm | 40000x | SE | 19.54 mm | 3.0 | 20.0 kV | SIO Analytical Facility |

FIG.2f

| HFW | Mag | Sig | WD | Spot | HV | 1 mm |
|---|---|---|---|---|---|---|
| 5.12 mm | 50x | SE | 19.46 mm | 3.0 | 20.0 kV | SIO Analytical Facility |

FIG.3a

| HFW | Mag | Sig | WD | Spot | HV | 100 μm |
|---|---|---|---|---|---|---|
| 0.51 mm | 500x | SE | 19.50 mm | 3.0 | 20.0 kV | SIO Analytical Facility |

FIG.3b

| HFW | Mag | Sig | WD | Spot | HV | 1 μm |
|---|---|---|---|---|---|---|
| 6.40 mm | 40000x | SE | 19.49 mm | 3.0 | 20.0 kV | SIO Analytical Facility |

FIG.3c

| HFW | Mag | Sig | WD | Spot | HV | 200 µm |
|---------|------|-----|----------|------|---------|------------------------|
| 0.85 mm | 300x | SE  | 19.13 mm | 3.0  | 20.0 kV | SIO Analytical Facility |

FIG.4b

| HFW | Mag | Sig | WD | Spot | HV | 1 μm |
|---|---|---|---|---|---|---|
| 6.40 μm | 40000x | SE | 19.11 mm | 3.0 | 20.0 kV | SIO Analytical Facility |

FIG.4c

| HFW | Mag | Sig | WD | Spot | HV | 200 μm |
|---------|------|-----|----------|------|----------|------------------------|
| 0.85 mm | 300x | SE | 58.97 mm | 3.0 | 20.0 kV | SIO Analytical Facility |

## FIG.4e

| HFW | Mag | Sig | WD | Spot | HV | 20 μm |
|---------|-------|-----|----------|------|----------|------------------------|
| 0.13 mm | 2000x | SE | 59.39 mm | 3.0 | 20.0 kV | SIO Analytical Facility |

## FIG.4f

FIG.5

FIG.6

32

30

# FIG.7
## PRIOR ART

FIG.8
PRIOR ART

FIG.9

FIG.10

FIG.11

Normalized Compression of MicroStat

FIG.12

Time (s)

FIG.13

EP 1 986 706 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03084582 A

- US 20030059371 A